# EUROPEAN PATENT APPLICATION

(11) **EP 4 410 985 A1**
(43) Date of publication of application: **07.08.2024**
(21) Application number: 23155120.1
(22) Date of filing: 06.02.2023
(51) Int. Cl.: C12N 15/82

(54) **A METHOD OF ECOLOGICAL STEM CELL DEVELOPMENT THAT STIMULATES GENE SHUFFLING AND HYBRID VIGOUR**

(71) Applicant: VRM International Pty Ltd, 4818 Bohle Queensland (AU)
(72) Inventor: Bellamy, Kenneth Michael, Bohle, 4818 (AU)
(74) Representative: LifeTech IP Spies & Behrndt Patentanwälte PartG mbB

(57) **Abstract**

A method of ecological stem cell development that stimulates gene shuffling comprising amending a growing site with one or more catalysts to obtain an amended growing site, wherein the one or more catalysts comprise a source of, and/or a substrate produced by and which stimulates the activity of, one or more prokaryotic organisms, and contacting one or more members of a Protostomia clade with a soil sample from the amended growing site, wherein an exchange of genetic material occurs between the prokaryotic organisms and the one or more members of the Protostomia clade.

## Description

### TECHNICAL FIELD

The present invention relates to a method of ecological stem cell development that stimulates gene shuffling. In particular, the present invention relates to a method of ecological stem cell development that involves amending a site with a catalyst which stimulates the activity of one or more prokaryotic organisms in the site to stimulate gene shuffling between organisms therein.

### BACKGROUND

The ever-increasing world population and the dwindling supply of arable land available for agriculture fuels research towards increasing the efficiency of agriculture. Conventional means for crop and horticultural improvements utilise selective breeding techniques (such as hybridisation or genetic engineering) to create plants having desirable traits (such as disease or insect resistance, matures faster, yield, or produces fruit of desired colour, taste or shape).

However, these techniques have several drawbacks, namely that these techniques are typically labour intensive and result in plants that often contain heterogeneous genetic components that may not always result in the desirable trait being passed on from parent plants.

While hybridisation produces organisms which have specific desirable traits and improved uniformity, this process may limit heterosis or hybrid vigour due to interbreeding and reduced genetic diversity. In addition, the seed produced from hybrid plants may be sterile, or not be true to type.

Further, there are concerns about health risks and environmental safety of genetically modified organisms. For example, while genetic modification of plants to express a Bt toxin has improved insect resistance in the plant, the action of the toxin is non-discriminatory causing high mortality rates in non-target species. In addition, as bacterial antibiotic resistance genes may be used as a marker gene during genetic engineering, there are concerns that these genes may be transferred to bacteria creating antibiotic-resistant strains.

It will be clearly understood that, if a prior art publication is referred to herein, this reference does not constitute an admission that the publication forms part of the common general knowledge in the art in Australia or in any other country.

### SUMMARY OF INVENTION

Embodiments of the present invention provide a method for relates to a method of ecological stem cell development that stimulates gene shuffling, which may at least partially address one or more of the problems or deficiencies mentioned above or which may provide the public with a useful or commercial choice.

As used therein, the term "in situ gene (or genetic) shuffling" designates any method of treating microorganisms contained in a sample to stimulate gene shuffling, i.e., the exchange (and/or reorganization) of genetic material.

As used herein, the term "contacting" is intended to refer to placement in direct physical association. For example, contacting can occur with a soil sample from the amended growing site and plants and/or plant parts (such as foliage, stem, seedlings, roots and/or seeds). Contacting can also occur with one or more microorganisms in the soil sample from the amended growing site (including any microbial metabolites or end products) and plants and/or plant parts.

The term "catalyst" as used herein is broadly defined as a substance that produces or generates a reaction regardless of whether it undergoes a change itself.

The term "amendment" or "amending" as used herein is broadly defined as a process or action that leads to a change in the condition of the land, including a physical change, a chemical change, a biological change, or any suitable combination thereof.

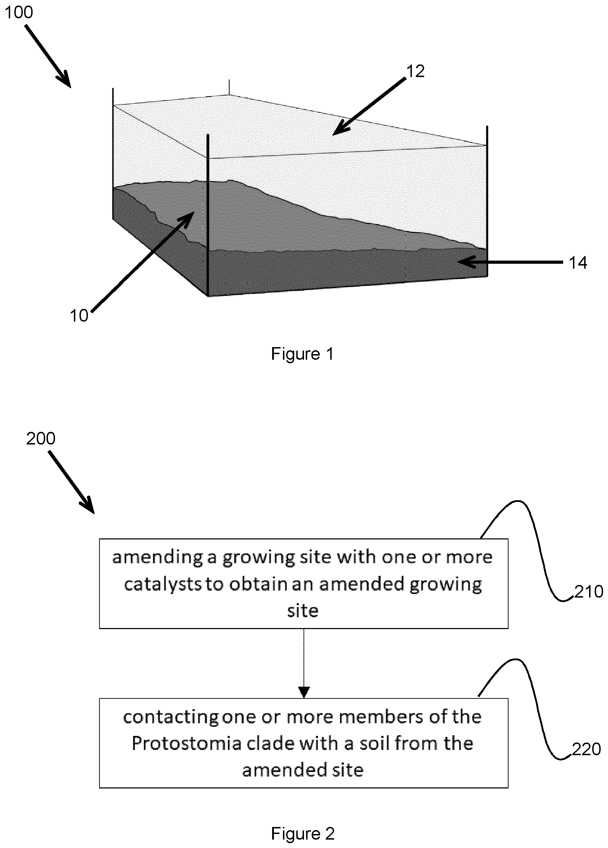

The term "growing site" as used herein, and with reference to Figure 1, is broadly defined as a three-dimensional space which includes the surface of a soil, the contiguous atmosphere above the soil and the three-dimensional area of the soil below the surface of the growth media. Preferably, the growing site may be defined as being an area which includes the surface of the soil, the contiguous atmosphere immediately above the soil to a height above the surface of the soil of approximately 1 metre and the three-dimensional area of the soil to a depth of about 100mm below the surface of the growth media.

For instance, the growing site may be arable land or non-arable land. The growing site may be pasturable land, meadows, grassland, agricultural land, farmland, orchards, plantations, forests, bush or scrub land, park land, residential land, golf courses, athletics fields, race courses, wetlands, water courses and bodies, land-based aquaculture facilities, rehabilitation sites, remediation sites, restoration site, revegetation site, fire-affected sites, mine sites, landfill, waste dumps, commercial composting facilities, on-farm composting facilities, or the like.

For instance, the growing site to be amended may be a container for growing plants and/or plant parts comprising a growth media therein. For instance, the container may be a pot, a bucket, a barrel, a tank, an intermediate bulk container (IBC), a greenhouse, or the like.

While the present invention may be discussed in connection with a "soil" it is not intended to be limited solely to a soil. Instead, the present invention may include any suitable growth media, such as a soil, clay, sand, vermiculite, perlite, coir, potting mix, composted bark, decomposed granite, sphagnum peat moss, straw, etc., that fall within the scope of the present invention.

According to a first aspect of the present invention, there is provided a method of ecological stem cell development that stimulates gene shuffling comprising:
amending a growing site with one or more catalysts to obtain an amended growing site, wherein the one or more catalysts comprise a source of, and/or a substrate produced by and which stimulates the activity of, one or more prokaryotic organisms, and
contacting one or more members of a Protostomia clade with a soil sample from the amended growing site,
wherein an exchange of genetic material occurs between the one or more prokaryotic organisms and the one or more members of the Protostomia clade.

According to a second aspect of the present invention, there is provided a method of ecological stem cell development that stimulates gene shuffling comprising:
amending a growing site with one or more catalysts to obtain an amended growing site, wherein the one or more catalysts comprise a source of, and/or a substrate produced by and which stimulates the activity of, one or more prokaryotic organisms, and
contacting a plant and/or plant part with a soil sample from the amended growing site,
wherein an exchange of genetic material occurs between the one or more prokaryotic organisms and the plant and/or plant part.

According to a third aspect of the present invention, there is provided an amended growing site when obtained according to the methods of the first or second aspects.

According to a fourth aspect of the present invention, there is provided a naturally genetically strengthened prostomial when obtained according to the method of the first aspect.

According to a fifth aspect of the present invention, there is provided a naturally genetically strengthened plant and/or plant part when obtained according to the method of the second aspect.

It will be understood that the term "naturally genetically strengthened" refers to improvements to the genes or genetics of the prostomial or plant and/or plant part that occur as the result of gene shuffling and contact between organisms resulting in genetic interface.

By naturally genetically strengthening a plant and/or plant part, it is envisaged that the plant or plant/part may be provided with improved disease or insect resistance, faster maturation rates, improved yield, or improved colour, taste or shape of the fruit and/or flower etc. produced by the plant. Similarly, by naturally genetically strengthening a prostomial, the properties of the soil (such as texture, structure, bulk density, porosity, consistency, temperature, colour and/or resistivity) in which the prostomial is located may be altered in such a manner as to create a soil environment in which plant growth properties (such as improved disease or insect resistance, faster maturation rates, improved yield, or improved colour, taste or shape of the fruit and/or flower etc. produced by the plant) may be improved.

Advantageously, amending a growing site with a catalyst which stimulates the activity of one or more prokaryotic organisms in the amended growing site stimulates gene shuffling between organisms in the amended growing site. In addition, exchange of genetic material between the prokaryotic organisms in the amended growing site with a plant and/or plant part or one or more members of a Protostomia clade may increase heterosis or hybrid vigour in the plant and/or plant part or the one or more members of the Protostomia clade. Beneficially, introducing a diverse prokaryotic community fosters and facilitates hybrid vigour without cross-breeding organisms cultivated in the amended growing site, improving the genetic strength of the food supply without introducing new hybrid or genetically modified organisms. In addition, the exchange of genetic material between the prokaryotic organisms and seeds harvested from a sterile/mule plant may result in a plant which has the ability to self-pollinate and/or produce viable seeded fruits.

As indicated, the method of ecological stem cell development that stimulates gene shuffling comprising comprises amending a growing site with one or more catalysts to obtain an amended growing site, wherein the one or more catalysts comprise a source of, and/or a substrate produced by and which stimulates the activity of, one or more prokaryotic organisms.

While the present invention is described in terms of one or more prokaryotic organisms, it is envisaged that the present invention may stimulate the activity of a plurality of prokaryotic organisms. In this way, a pool of stimulated prokaryotic organisms may be generated by the present invention. Preferably, the pool of stimulated prokaryotic organisms comprises two or more varieties of prokaryotic organisms.

Any suitable type of growing site may be amended. For instance, the growing site may be arable land, non-arable land, pasturable land, meadows, grassland, agricultural land, farmland, orchards, plantations, forests, bush or scrub land, park land, residential land, golf courses, athletics fields, race courses, wetlands, water courses and bodies, land-based aquaculture facilities, rehabilitation sites, remediation sites, restoration site, revegetation site, fire-affected sites, mine sites, landfill, waste dumps, commercial composting facilities, on-farm composting facilities, or the like.

For instance, the growing site to be amended may be a container for growing plants and/or plant parts comprising a growth media therein. For instance, the container may be a pot, a bucket, a barrel, a tank, an intermediate bulk container (IBC), a greenhouse, or the like.

As used herein, the term "stimulates the activity of one or more prokaryotic organisms" means that the level of activity level of the prokaryotic organisms is increased when the one or more catalysts are applied to the growing site.

Any suitable catalyst may be used. Generally, the catalysts may comprise a source of, and/or a substrate produced by, and which stimulates the activity of, one or more prokaryotic organisms. For instance, the catalyst may provide a substate which stimulates the activity of one or more species of Archaea, one or more species of bacteria, or any suitable combination thereof. The prokaryotic organism may be anerobic, aerobic, autotrophic, heterotrophic, phototrophic, chemotrophic, photosynthetic, or any suitable combination thereof.

Generally, the one or more catalysts provide a substrate which may stimulate the activity of low temperature fermentation microorganisms. As used herein, low temperature fermentation microorganisms refer to microorganisms at which the optimum conditions for fermentation occurs at temperatures of below 30°C, more preferably below 28°C, more preferably below 25°C and more preferably below 20°C. In a preferred embodiment of the invention, the catalyst may comprise a humified soil prepared from the continuous fermentation of an organic material. In an embodiment of the invention, the first catalyst may comprise a humified soil prepared from the continuous fermentation of an organic material, wherein the continuous fermentation comprises a source of and provides a habitat for the sustained activity of low temperature fermentation microorganisms. In a preferred embodiment of the invention, the catalyst may comprise a humified soil prepared from the continuous fermentation of an organic material, wherein the humified soil comprises a viable source of and/or substrates produced by and which stimulate the activity of at least one of an aerobic microorganism, an anaerobic microorganism and a photosynthetic microorganism. Preferably, the catalyst may comprise a humified soil prepared from the continuous fermentation of an organic material, wherein the humified soil comprises a source of and/or substrates produced by, and which stimulate the activity of heterotrophic photosynthetic bacteria and/or prokaryotic organisms including either one of Archaea or bacteria. In an embodiment of the invention, the first catalyst may be an output product produced by the method and/or system described in Australian patent no. 2014250680, the disclosure of which is incorporated herein by reference. In some embodiments, the catalyst may stimulate the activity of at least one of an aerobic microorganism, an anaerobic microorganism, and a photosynthetic microorganism. Preferably, the catalyst may stimulate the activity of a heterotrophic photosynthetic bacteria and/or prokaryotic organism including either one of Archaea or bacteria.

In other embodiments of the invention, the catalyst may comprise a liquid fertiliser. In a preferred embodiment of the invention, the catalyst may comprise a liquid fertiliser prepared from the continuous fermentation of an organic material. In an embodiment of the invention, the catalyst may comprise a liquid fertiliser prepared from the continuous fermentation of an organic material, wherein the liquid fertiliser may be a reconstituted microbial substrate. In an embodiment of the invention, the second catalyst comprises a liquid fertiliser prepared from the continuous fermentation of an organic material, wherein the continuous fermentation comprises a source and a habitat for the activity of low temperature fermentation microorganisms. In a preferred embodiment of the invention, the second catalyst comprises a liquid fertiliser prepared from the continuous fermentation of an organic material, wherein the liquid fertiliser comprises a viable source of and/or substrates produced by and which stimulate the activity of at least one of an aerobic microorganism, an anaerobic microorganism, a heterotrophic microorganism and a photosynthetic microorganism. Preferably, the second catalyst comprises a liquid fertiliser prepared from the continuous fermentation of an organic material, wherein the liquid fertiliser comprises a source of and/or substrates produced by and which stimulate the activity of heterotrophic photosynthetic bacteria and/or prokaryotic organisms including either one of Archaea or bacteria. In an embodiment of the invention, the second catalyst may be an output product produced by the method and/or system described in Australian patent no. 2012283757, the disclosure of which is incorporated herein by reference.

In some embodiments, the catalyst may stimulate the activity of one or more prokaryotic organisms, such as heterotrophic photosynthetic bacteria, lactobacillus species, yeasts, actinomycetes species, Nocardia species, ray fungi, plankton, chemotrophic bacteria, or any suitable combination thereof.

In some embodiments, the catalyst may comprise a liquor, a fertiliser (and particularly a biofertilizer) or other high value organic material, a humus or humified soil, an incubated culture, a collected substrate for energy generation, or the like.

In use, it is envisaged that amending the growing site with the catalyst may stimulate the activity of one or more prokaryotic organisms in the soil sample in the growing site, stimulating gene shuffling between organisms in the amended growing site. For instance, the prokaryotic organism may comprise one or more species of Archaea, one or more species of bacteria, or any suitable combination thereof. The prokaryotic organism may be anerobic, aerobic, autotrophic, heterotrophic, phototrophic, chemotrophic, photosynthetic, or any suitable combination thereof. In a preferred embodiment of the invention, the prokaryotic organisms may include purple non-sulphur producing heterotrophic photosynthetic bacteria, Lactobacillus species, yeasts, Actinomycetes species, Nocardia species, a ray fungi, plankton, chemotrophic bacteria, or an suitable combination thereof. Advantageously, creating a diverse prokaryotic community in the site fosters and facilitates heterosis or hybrid vigour without cross-breeding the organisms, improving the genetic strength of the food supply without introducing new hybrid or genetically modified organisms.

As indicated, the method of ecological stem cell development that stimulates gene shuffling comprising may comprise contacting one or more members of a Protostomia clade with a soil sample from the amended growing site. Any suitable period of contact may be required in order to effect gene shuffling, and it will be understood that the period of time may be dependent on a number of factors, such as, but not limited to, the specific members of the Protostomia clade, the properties of the soil, the nature of the amended growing site and so on. In some embodiments of the invention, the period of contact may be between one minute and six months. More preferably, the period of contact may be between 15 minutes and one month. Yet more preferably, the period of contact may be between 30 minutes and one week. Still more preferably, the period of contact may be between one hour and one day.

The one or more members of the Protostomia clade may comprise members of Nematoda, Annelida, Mollusca, Plathyhelminthes, Tactopoda clade, or any suitable combination thereof. However, a person skilled in the art will appreciate that any organism present in the amended growing site may be contacted by the soil sample from the amended growing site.

The one or more members of the Protostomia clade may be contacted with the soil sample from the amended growing site in any suitable manner. Generally, the manner of contact may be sufficient to enable an exchange of genetic material between the prokaryotic organisms in the soil sample from the amended growing site and the one or more members of the Protostomia clade.

In some embodiments, contacting the one or more members of the Protostomia clade with a soil sample from the amended growing site includes cultivating the one or more members of the Protostomia clade in the soil sample.

In some embodiments, contacting the one or more members of the Protostomia clade with a soil sample from the amended growing site includes growing one or more treated members of the Protostomia clade.

For example, the soil sample from the amended growing site may be used as a growth media to grow one or more members of the Protostomia clade before the one or more treated members of the Protostomia clade are transferred to a growing site. In this instance, the growing site may be an amended growing site or may be an unamended growing site.

The exchange of genetic material may occur between the prokaryotic organisms and the one or more members of the Protostomia clade may occur in any suitable manner. For instance, the exchange of genetic material may occur directly between the prokaryotic organisms and the one or more members of the Protostomia clade, indirectly via an exchange of genetic material between the prokaryotic organisms and a symbiotic microorganism associated with the one or more members of the Protostomia clade, or by incorporation of the prokaryotic organism into the gut flora of the one or more members of the *Protostomia* clade.

The method of stimulating gene shuffling may comprise contacting a plant and/or plant part with the soil sample from the amended growing site. Any suitable period of contact may be required in order to effect gene shuffling, and it will be understood that the period of time may be dependent on a number of factors, such as, but not limited to, the specific plant and/or plant part, the properties of the soil, the nature of the amended growing site and so on. In some embodiments of the invention, the period of contact may be between one minute and six months. More preferably, the period of contact may be between 15 minutes and one month. Yet more preferably, the period of contact may be between 30 minutes and one week. Still more preferably, the period of contact may be between one hour and one day.

Any suitable type of plant may be used. For instance, the plant may be a food plant, a non-food plant, a medicinal plant, an ornamental plant, an aquatic plant, or the like.

Any suitable type of plant parts may be used. Preferably, however, the plants parts may be any suitable organism that performs green plant photosynthesis. For instance, the plant parts may comprise foliage, stems, seedlings, roots, algae, cyanobacteria and/or seeds.

The plants and/or plant parts may be contacted with the soil sample from the amended growing site in any suitable manner. Generally, the manner of contact may be sufficient to enable an exchange of genetic material between the prokaryotic organisms in the soil sample from the amended growing site and the plant and/or plant part.

In some embodiments, contacting a plant and/or plant part with a soil sample from the amended growing site includes cultivating a plant and/or plant part in the soil sample. In other embodiments, contacting a plant and/or plant part with a soil sample from the amended growing site includes growing a treated plant and/or plant part.

For example, the soil sample from the amended growing site may be used as a growth media to propagate a plant and/or plant part before the treated plant and/or plant part is transferred to a growing site for growing. In this instance, the growing site may be an amended growing site or may be an unamended growing site.

In some embodiments, the soil sample from the amended growing site may comprise one or more treated members of the Protostomia clade.

The exchange of genetic material may occur between the prokaryotic organisms and the plant and/or plant part in any suitable manner.

For instance, the exchange of genetic material may occur directly between the prokaryotic organisms and the plant and/or plant part, indirectly via an exchange of genetic material between the prokaryotic organisms and a symbiotic microorganism associated with the plant and/or plant part, or by incorporation of the prokaryotic organism into a colony associated with the plant and/or plant part.

In some embodiments, the genetic material exchanged between the prokaryotic organisms and the plant and/or plant part may comprise genetic material exchanged between the prokaryotic organisms and the one or more members of the Protostomia clade.

In some embodiments, the method of stimulating gene shuffling comprises subjecting the soil sample from the amended growing site to a stimulus.

Any suitable stimuli may be used. For instance, the stimuli may be a chemical stimulus, electrical stimuli, auditory or vibrotactile stimuli, electromagnetic radiation, aroma, temperature, an interaction with a human, or the like.

Generally, the stimuli may be sufficient to enhance the interaction between the prokaryotic organisms in an amended growing site with a plant and/or plant part or one or more members of the Protostomia clade, and/or improve the exchange of genetic material between the prokaryotic organisms in an amended growing site with a plant and/or plant part or one or more members of the Protostomia clade.

In some embodiments, the stimuli may stimulate a positive feedback loop.

In use, it is envisaged that the method of the present invention may be used to exchange genetic material between the prokaryotic organisms and seeds harvested from a sterile/mule plant may result in a plant which has the ability to self-pollinate and/or produce viable seeded fruits.

In a preferred embodiment of the invention, the method of the invention may be performed within a preferred range of soil pH values. While any suitable soil pH values are envisaged, it is preferred that the method may be performed at a soil pH between approximately 1 and 10. More preferably, the method may be performed at a soil pH between approximately 2 and 8. More preferably, the method may be performed at a soil pH of between approximately 3 and 6. Still more preferably, the method may be performed at a soil pH of between approximately 4.5 and 5.5.

The soil pH may be adjusted to within the preferred range using any suitable technique. For instance, the soil pH may be adjusted by the addition of one or more chemical substances. Any suitable chemical substance may be used, although it will be understood that an acidic substance may be added if it is desired to lower the pH, while a basic substance may be added if it is desired to raise the soil pH. Preferably, the chemical substance does not have an adverse effect on the method (such as the rate of the method or the like) or the organisms involved in the method.

It is envisaged, however, that the pH of the soil may be an indicator of other factors or processes within the soil. For instance, the soil pH may provide an indicator of the balancing of hydrogen ions within the soil, with the correct management of hydrogen ions in the soil leading to improved biological activity within the soil. Thus, in a preferred embodiment, pH of the soil may be adjusted as a function of the balancing of hydrogen ion within the soil. Specifically, by amending the soil with one or more catalysts, the on-going capacity of the soil to balance hydrogen compounds may be facilitated. In turn, the balancing of hydrogen compounds within the soil underpins the acidity, alkalinity or sodic contamination of the soil.

In a preferred embodiment of the invention, amending the soil with a first catalyst and a second catalyst may facilitate the transfer of a biological energy generation mechanism to the soil. In a preferred embodiment, the biological energy generation mechanism comprises the capture of solar energy outside the spectral range used by plants by one or more photosynthetic bacteria and the subsequent storage of the captured energy as an organic molecule. It will be understood that the solar energy is initially stored as chemical energy transfer compounds (such as ATP and NADPH), which are subsequently used by photosynthetic organisms to build other compounds rich in hydrogen (such as carbohydrates, proteins and water) which act as both energy storage compounds and nutrient storage compounds.

It will be understood that the biological energy generation process may trigger a nutrient accumulation process, including, but not limited to, nitrogen and carbon sequestration, and subsequently results in the formation of a humified soil, wherein the humified soil may be an energy storage compound and a nutrient storage compound which is not negatively impacted by either an excess or a deficit of available hydrogen ions or by sodic contamination. In use, it is envisaged that the improved nutrient storage and water storage capacity resulting from the development of a humified soil may assist in the absorption and management of contaminants, including contamination arising from an imbalance in hydrogen ions and/or sodicity.

In use, it is envisaged that the method for amending the soil to balance hydrogen ions may support the development of the local microbiome or ecosystem, such that there may be sufficient stored energy to support the structures needed for a healthy microbiome (such as bacteria, Archaea, viruses, fungi, protozoa, and the like) and subsequently the organisms interacting with the microbiome (such as humans, plants, animals, earthworms, insects, and the like). In use, it is envisaged that sustained energy generation may improve the ability of the soil microbiome to recover from and/or resist disease. Advantageously, this natural restoration may result in the regeneration of indigenous flora and/or fauna in the site since these are dependent on the environmental conditions and microbiome unique to the site.

More specifically, biological energy is generated in the matrix of biological energy generation points by capturing solar energy outside the spectral range used by plants by one or more photosynthetic bacteria and subsequently storing the captured energy as an organic molecule in the soil, the organic molecule being used by photosynthetic organisms in the soil to build hydrogen-rich compounds.

In some embodiments of the invention, the pH of the soil may be adjusted by balancing the acidity or alkalinity of the soil. This may be achieved using any suitable technique, such as, but not limited to, balancing the hydrogen cycle, buffering the soil pH, facilitating cation exchange, or any suitable combination thereof.

In a preferred embodiment of the invention, the method of the invention may be performed within a preferred range of soil moisture values. While any suitable soil moisture values are envisaged, it is preferred that the method may be performed at a soil moisture within the range of 1% w/w and 99% w/w. More preferably, the method may be performed at a soil moisture within the range of 2% w/w and 90% w/w. More preferably, the method may be performed at a soil moisture within the range of 3% w/w and 80% w/w. More preferably, the method may be performed at a soil moisture within the range of 4% w/w and 75% w/w. More preferably, the method may be performed at a soil moisture within the range of 5% w/w and 70% w/w.

The soil moisture may be adjusted to within the preferred range using any suitable technique. For instance, the soil moisture maybe adjusted by the addition of water, or by drying the soil to remove moisture therefrom.

In other embodiments of the invention, the soil moisture may be adjusted through hydrosynthesis, and in particular, biological hydrosynthesis generated within the soil. Biological hydrosynthesis may be generated using any suitable technique, although in some embodiments of the invention, it is envisaged that biological hydrosynthesis may be generated through one or more amendments of the soil using one or more catalysts to construct a matrix of biological energy generation points.

In a particular embodiment, biological hydrosynthesis in the soil may be achieved by undertaking a primary amendment of the soil with a first catalyst and a second catalyst, wherein the first catalyst and the second catalyst are applied to only a first portion of the soil wherein the first catalyst comprises humified soil prepared from continuous fermentation of an organic material and the second catalyst comprises a liquid fertiliser prepared from continuous fermentation of an organic material, and wherein the first portion of the soil is about 5% of the soil by area, subsequently undertaking a secondary amendment of the soil with the first catalyst and the second catalyst, wherein the first catalyst and the second catalyst are applied to only a second portion of the soil wherein the second portion is about 20% of the soil by area and wherein the second portion of the soil includes the first portion of the soil, and subsequently undertaking a tertiary amendment of the soil with the first catalyst and the second catalyst, wherein the first catalyst and the second catalyst are applied to only a third portion of the soil wherein the third portion is about 75% of the soil by area and wherein the third portion of the soil includes the second portion of the soil. Preferably, the method of biological hydrosynthesis described herein consists of only primary, secondary and tertiary amendments, such that a total of 75% of the soil is treated.

In a preferred embodiment of the invention, the method of the invention may be performed within a preferred range of soil temperatures. While any suitable soil temperatures are envisaged, it is preferred that the method may be performed at a soil temperature within the range of 10°C and 40°C. More preferably, the method may be performed at a soil temperature within the range of 15°C and 35°C. More preferably, the method may be performed at a soil temperature within the range of 20°C and 30°C. More preferably, the method may be performed at a soil temperature within the range of 22°C and 28°C.

It has been found that, when the soil temperature is maintained within the preferred range, the development of fungi within the soil may be promoted.

The temperature of the soil may be maintained within the preferred range using any suitable technique. However, it will be understood that the soil temperature is not adjusted through chemical amendment of the soil or through external irrigation. Instead, it is envisaged that the soil temperature may be adjusted (and, preferably, maintained within the preferred range) through a combination of the endothermic nature of the hydrosynthesis process occurring in the soil, and the exothermic nature of water consumption that occurs during photosynthesis.

In a preferred embodiment of the invention, the soil pH, the soil moisture and the soil temperature may all be adjusted to and/or maintained within the preferred ranges. It has been found that, but maintaining the soil pH, the soil moisture and the soil temperature within the preferred ranges, the effect produced by the method of the present invention may be optimised.

It is envisaged that the method of the present invention is configured to specifically stimulate and maintain a pool of active, relatively highly motile prokaryotic organisms over an extended period of time. It is envisaged that, as a result of the method of the present invention, the prokaryotic organisms are specifically provided with the capacity to manipulate genetic material.

The ability to maintain the properties of the prokaryotic organisms over an extended period of time is achieved through the amendments made to the growing site in the method of the present invention. It would not be possible to maintain the properties of the prokaryotic organisms over an extended period of time without the specific steps taken in the method of the present invention.

As stated above, the prokaryotic organisms stimulated by the present invention are highly motile. It is envisaged that this motility is facilitated by the capacity of individual organisms, as well as the collective pool of organisms, to generate and maintain a reserve of moisture within the soil through which the prokaryotic organisms move. Thus, the stimulation of the prokaryotic organisms in the present invention results in hydrosynthesis within the soil.

While the invention has been described in terms of the stimulation of one or more prokaryotic organisms, it is envisaged that improved results may be achieved by the net total activity of a plurality of organisms, and particularly the net total activity of a plurality of diverse organisms. It is envisaged that the organised may act in a sustained pool, and improved results may be achieved as a result of the activity of the sustained pool of organisms, rather than through the activity of any particular individual organism.

The present invention provides numerous advantages over the prior art. For instance, the present invention results in prostomials, plants and/or plant parts that are naturally genetically strengthened, meaning that concerns regarding the health risks and environmental safety of artificially genetically modified organisms may be reduced or eliminated. Further, the method of the present invention produces plants or plant/parts provided with improved disease or insect resistance, faster maturation rates, improved yield, or improved colour, taste or shape of the fruit and/or flower etc. produced by the plant. Similarly, the present invention produces soil having improved properties (such as texture, structure, bulk density, porosity, consistency, temperature, colour and/or resistivity) that create a soil environment in which plant growth properties (such as improved disease or insect resistance, faster maturation rates, improved yield, or improved colour, taste or shape of the fruit and/or flower etc. produced by the plant) are improved.

Any of the features described herein can be combined in any combination with any one or more of the other features described herein within the scope of the invention.

The reference to any prior art in this specification is not, and should not be taken as an acknowledgement or any form of suggestion that the prior art forms part of the common general knowledge.

### BRIEF DESCRIPTION OF DRAWINGS

Preferred features, embodiments and variations of the invention may be discerned from the following Detailed Description which provides sufficient information for those skilled in the art to perform the invention. The Detailed Description is not to be regarded as limiting the scope of the preceding Summary of Invention in any way. The Detailed Description will make reference to a number of drawings as follows:
Figure 1 illustrates a three-dimensional space defined as a site according to an embodiment of the invention; and
Figure 2 illustrates a flowchart showing steps in a method of ecological stem cell development that stimulates gene shuffling comprising according to an embodiment of the invention.

### DETAILED DESCRIPTION

In Figure 1, a three-dimensional space defined as a growing site 100 according to an embodiment of the invention is illustrated. Growing site 100 may be defined as being a three-dimensional space which includes the surface of the soil 10, the contiguous atmosphere above the soil 12 and the three-dimensional area of the soil below the surface of the soil 14.

In use it is envisaged that one or more catalysts (not shown) may be applied to at least a portion of the growing site 100, wherein the at least a portion of the growing site 100 may include the surface of the soil 10, the contiguous atmosphere above the soil 12 and the three-dimensional area of the soil below the surface of the soil 14.

For instance, the catalysts (not shown) may be applied to the growing site 100 as a spray, wherein the liquid droplets may be dispersed through the contiguous atmosphere above the soil 12 and onto the soil surface 10 where they subsequently migrate into or, are tilled into, the soil body 14. In this way, it is envisaged that applying the one or more catalysts to at least a portion of the growing site stimulates the activity of one or more prokaryotic organisms, stimulating gene shuffling between organisms in the growing site.

A method 200 of ecological stem cell development that stimulates gene shuffling comprising is now described in detail with reference to Figure 2.

At step 210, a growing site is amended by applying one or more catalysts to the growing site.

Generally, the catalysts may comprise a source of, and/or a substrate produced by and which stimulates the activity of, one or more prokaryotic organisms.

In use, it is envisaged that amending the growing site with the catalyst may stimulate the activity of one or more prokaryotic organisms in the soil in the growing site, stimulating gene shuffling between organisms in the growing site.

At step 220, one or more members of the Protostomia clade may be contacted with a soil sample from the amended growing site. In some embodiments, the one or more members of the Protostomia clade may be cultivated in the soil sample.

The contact between the prokaryotic organisms and the one or more members of the Protostomia clade causes an exchange of genetic material to occur.

In some embodiments, a plant and/or plant part may be contacted with the soil sample from the amended growing site, causing an exchange of genetic material to occur between the prokaryotic organisms in the soil sample and the plant and/or plant part.

In some embodiments, the plant and/or plant part may be cultivated in the soil sample.

In some embodiments, the soil sample from the amended growing site comprises one or more treated members of the Protostomia clade. In this instance, it will be understood that a treated member of the Protostomia clade may be formed from the exchange of genetic material between the prokaryotic organisms and the member of the Protostomia clade.

The soil sample from the amended growing site may be subjected to a chemical stimuli, electrical stimuli, auditory or vibrotactile stimuli, electromagnetic radiation, aroma, temperature, an interaction with a human, or the like. Preferably, the stimuli may stimulate a positive feedback loop.

In some embodiments, the stimuli may be an interaction with a human.

In the present specification and claims (if any), the word *'comprising'* and its derivatives including *'comprises'* and *'comprise'* include each of the stated integers but does not exclude the inclusion of one or more further integers.

Reference throughout this specification to *'one embodiment'* or *'an embodiment'* means that a particular feature, structure, or characteristic described in connection with the embodiment is included in at least one embodiment of the present invention. Thus, the appearance of the phrases *'in one embodiment'* or *'in an embodiment'* in various places throughout this specification are not necessarily all referring to the same embodiment. Furthermore, the particular features, structures, or characteristics may be combined in any suitable manner in one or more combinations.

In compliance with the statute, the invention has been described in language more or less specific to structural or methodical features. It is to be understood that the invention is not limited to specific features shown or described since the means herein described comprises preferred forms of putting the invention into effect. The invention is, therefore, claimed in any of its forms or modifications within the proper scope of the appended claims (if any) appropriately interpreted by those skilled in the art.

## Claims

1. A method of ecological stem cell development that stimulates gene shuffling comprising:
amending a growing site with one or more catalysts to obtain an amended growing site, wherein the one or more catalysts comprise a source of, and/or a substrate produced by, and which stimulates the activity of, one or more prokaryotic organisms; and
contacting one or more members of a Protostomia clade with a soil sample from the amended growing site,
wherein an exchange of genetic material occurs between the one or more prokaryotic organisms and the one or more members of the Protostomia clade.

2. A method of ecological stem cell development that stimulates gene shuffling comprising:
amending a growing site with one or more catalysts to obtain an amended growing site, wherein the one or more catalysts comprise a source of, and/or a substrate produced by, and which stimulates the activity of, one or more prokaryotic organisms; and
contacting a plant and/or plant part with the soil sample from the amended growing site, wherein an exchange of genetic material occurs between the one or more prokaryotic organisms and the plant and/or plant part.

3. A method according to claim 1 or claim 2 wherein a first of the one or more catalysts provides a substrate that stimulates the activity of low temperature fermentation microorganisms.

4. A method according to claim 3 wherein the first of the one or more catalysts comprises a humified soil prepared from the continuous fermentation of an organic material, wherein the continuous fermentation comprises a source of, and provides a habitat for, sustained activity of the low temperature fermentation microorganisms.

5. A method according to claim 1 or claim 2 wherein a second of the one or more catalysts comprises a liquid fertiliser prepared from continuous fermentation of an organic material, wherein the continuous fermentation comprises a source of, and a habitat for, the activity of low temperature fermentation microorganisms.

6. A method according to any one of the preceding claims wherein the one or more prokaryotic organisms comprise one or more species of Archaea and/or one or more species of bacteria.

7. A method according to any one of the preceding claims wherein the one or more prokaryotic organisms include purple non-sulphur producing heterotrophic photosynthetic bacteria, Lactobacillus species, yeasts, Actinomycetes species, Nocardia species, a ray fungi, plankton and/or chemotrophic bacteria.

8. A method according to claim 1 wherein the one or more members of the Protostomia clade may comprise members of Nematoda, Annelida, Mollusca, Plathyhelminthes and/or Tactopoda clade.

9. A method according to claim 1 or claim 8 wherein contacting one or more members of the Protostomia clade with the soil sample from the amended growing site includes cultivating the one or more members of the Protostomia clade in the soil sample.

10. A method according to claim 1 wherein the exchange of genetic material between the prokaryotic organisms and the one or more members of the Protostomia clade occurs directly between the prokaryotic organisms and the one or more members of the Protostomia clade, indirectly via an exchange of genetic material between the prokaryotic organisms and a symbiotic microorganism associated with the one or more members of the Protostomia clade, or by incorporation of the prokaryotic organism into gut flora of the one or more members of the Protostomia clade.

11. A method according to claim 2 wherein the plant part comprises foliage, stems, seedlings, roots, algae, cyanobacteria and/or seeds.

12. A method according to claim 2 or claim 11 wherein contacting the plant and/or plant part with the soil sample from the amended growing site includes cultivating the plant and/or plant part in the soil sample, or growing a treated plant and/or plant part.

13. A method according to claim 2 wherein the exchange of genetic material occurs directly between the prokaryotic organisms and the plant and/or plant part, indirectly via an exchange of genetic material between the prokaryotic organisms and a symbiotic microorganism associated with the plant and/or plant part, or by incorporation of the prokaryotic organism into a colony associated with the plant and/or plant part.

14. An amended growing site when obtained according to the method of claim 1 or claim 2.

15. A naturally genetically strengthened prostomial when obtained according to the method of claim 1, or a naturally genetically strengthened plant and/or plant part when obtained according to the method of claim 2.
